# DEMANDE DE BREVET EUROPEEN

(11) **EP 3 028 685 A1**
(43) Date de publication de la demande: **08.06.2016**
(21) Numéro de dépôt: 15003382.7
(22) Date de dépôt: 26.11.2015
(51) Int. Cl.: A61F 9/02

(54) **MASQUE AVEC ÉLÉMENT DE CONFORT**

(30) Priorité: 06.12.2014 FR 1402790
(71) Demandeur: Salomon S.A.S., 74370 Metz-Tessy (FR)
(72) Inventeur: Favre-Felix, Hervé, 74370 Villaz (FR); Donnadieu, Thierry, 74330 Sillingy (FR)

(57) **Abrégé**

Masque de protection oculaire (1) pour la pratique de sports en plein air comprenant :
- un écran de protection oculaire (3),
- un châssis (2) supportant l'écran,
- un élément de confort (5) solidaire du châssis, l'élément de confort comprenant une partie nasale (52) destinée à venir en contact avec une partie du nez de l'utilisateur et une partie frontale (51) destinée à venir en contact avec une partie du front de l'utilisateur.

Le matériau utilisé pour la partie nasale présente des propriétés mécaniques d'écrasement différentes du matériau utilisé pour la partie frontale de sorte que la course d'écrasement maximale du matériau de la partie nasale sollicité par un effort de compression déterminé est plus importante que la course d'écrasement du matériau de la partie frontale sollicité par le même effort de compression déterminé.

## Description

L'invention concerne les masques de protection oculaire, en particulier les masques de protection pour la pratique de sports en plein air, tels que le ski ou surf alpin, le vélo tout terrain ou le motocross.

Des masques de protection sont habituellement utilisés durant la pratique de certains sports de plein air pour protéger les yeux de l'utilisateur du vent, de l'eau, de la boue ou du rayonnement solaire.

Un tel masque comprend généralement un châssis supportant un écran de protection transparent et une sangle fixée de part et d'autre du châssis. La sangle permet de maintenir le masque sur le visage en entourant partiellement le crâne de l'utilisateur. Des éléments de confort sont habituellement ajoutés au châssis pour améliorer le confort du port du masque. Ces éléments de confort sont placés à l'interface entre le châssis et le visage. Ils forment souvent une bande de mousse entourant les yeux en prenant appui sur une partie du front, les tempes, les pommettes et une partie du nez de l'utilisateur.

Ces éléments de confort se caractérisent par des propriétés d'amortissement et de rugosité de la surface de contact. En effet, pour maintenir le masque au niveau des yeux, il faut exercer un effort de placage du châssis vers le visage, via la sangle. S'il n'y a pas d'éléments de confort, le contact du châssis plastique sur le visage n'est pas agréable. De plus, le châssis se déforme peu et ne permet pas une bonne adaptation aux différentes morphologies du visage. L'étanchéité à l'air du volume interne du masque n'est pas très bonne. Des courants d'air peuvent pénétrer dans ce volume ce qui est également désagréable pour les yeux. Pour pallier ces problèmes, on ajoute généralement une mousse de confort sur le châssis. Cette mousse apporte l'adaptation à la morphologie du visage, l'atténuation de la pression de contact, la douceur et l'isolation recherchée.

Le document US 2011/0289662 propose un élément de confort réalisé en mousse visqueuse. Ce matériau s'adapte bien à la morphologie dans la limite du débattement local possible de la mousse. Par ailleurs, c'est un matériau peu évident à mettre en forme.

Le document US 2013/0067626 présente un élément de confort multicouche dont l'épaisseur de l'élément est plus important dans les zones latérales que dans les zones centrales supérieure et inférieure. Cette construction permet un bon placage des zones latérales lorsque l'utilisateur porte un casque.

Les éléments de confort de l'art antérieur présentent sensiblement les mêmes caractéristiques d'écrasement sur le pourtour des yeux. Ils ont souvent une épaisseur sensiblement constante et peu épaisse, en tout cas, dans les zones de contact du front et du nez. Or, la morphologie du visage d'un individu à l'autre est très variable et en particulier, au niveau du nez. Ainsi, en fonction de la morphologie de l'utilisateur, l'adaptation morphologique sera sensiblement similaire dans la zone frontale et dans la zone nasale, ce qui n'est pas très confortable. Le nez peut alors subir une pression désagréable. De plus, l'enveloppement du nez peut s'avérer ne pas être très bon. L'étanchéité du masque peut être moins bonne d'un individu à l'autre du fait que la déformation de l'élément de confort au niveau de la zone nasale est généralement faible.

Le but de l'invention est de proposer un masque amélioré permettant un meilleur confort de portage.

Un but est notamment de mieux s'adapter aux différentes morphologies des individus.

Un autre but est de contrôler la répartition de la pression de maintien du masque sur le visage sur des zones définies.

Un autre but est de diminuer la pression exercée au niveau du nez.

Un autre but est d'améliorer l'étanchéité du volume intérieur du masque.

L'invention propose un masque de protection oculaire pour la pratique de sport en plein air comprenant :
- un écran de protection oculaire,
- un châssis supportant l'écran,
- un élément de confort solidaire du châssis, l'élément de confort comprenant une partie nasale destinée à venir en contact avec une partie du nez de l'utilisateur et une partie frontale destinée à venir en contact avec une partie du front de l'utilisateur.

Le masque est caractérisé par le fait que le matériau utilisé pour la partie nasale présente des propriétés mécaniques d'écrasement différentes du matériau utilisé pour la partie frontale de sorte que la course d'écrasement maximale du matériau de la partie nasale sollicité par un effort de compression déterminé est plus importante que la course d'écrasement du matériau de la partie frontale sollicité par le même effort de compression déterminé.

Grâce à cette construction, la partie nasale de l'élément de confort peut se déformer davantage que la partie frontale. Elle peut donc mieux s'adapter à la géométrie complexe du nez et exercer une plus faible pression au niveau des ailes du nez, zones sensibles. La respiration de l'utilisateur est peu perturbée car les narines restent pleinement ouvertes, les ailes du nez n'étant pas écrasées. En épousant mieux les courbes du nez, l'élément de confort améliore l'étanchéité dans cette zone.

Selon des aspects avantageux mais non obligatoires de l'invention, un tel masque peut incorporer une ou plusieurs des caractéristiques suivantes, prises dans toute combinaison techniquement admissible :
- Au moins un matériau constituant les parties nasale et/ou frontale est composite, le matériau comprenant alors plusieurs couches de matières différentes.
- Le matériau de la partie frontale et/ou le matériau de la partie nasale comprend successivement, à partir du châssis, une première couche de mousse d'un premier type et une seconde couche de mousse d'un deuxième type, la densité de la mousse de la première couche étant plus forte que la densité de la mousse de la seconde couche.
- L'épaisseur de la seconde couche de la partie nasale est supérieure à l'épaisseur de la seconde couche de la partie frontale.
- Les deux secondes couches sont constituées du même type de mousse.
- La densité de la mousse de la seconde couche de la partie nasale est inférieure à la densité de la mousse de la seconde couche de la partie frontale.
- La mousse de la première couche de la partie nasale est de nature différente de la mousse de la première couche de la partie frontale.
- L'épaisseur de la partie nasale est supérieure à l'épaisseur de la partie frontale.
- L'élément de confort comprend deux parties latérales, chaque partie latérale étant destinée à venir en contact avec la tempe et la pommette d'un côté du visage de l'utilisateur, chaque partie latérale reliant une extrémité de la partie frontale à une extrémité de la partie nasale.
- Le matériau utilisé pour les parties latérales présentant des propriétés mécaniques d'écrasement intermédiaire par rapport aux matériaux utilisés pour les parties nasale et frontale.
- Le nombre de couches de matière entre les différentes parties de l'élément de confort est différent.
- L'élément de confort comprend au moins deux pièces distinctes, une première pièce formant la partie nasale, la deuxième pièce formant la partie frontale.
- L'élément de confort comprend quatre pièces distinctes ; une partie frontale, une partie nasale et deux parties latérales.
- Au moins une partie de l'élément de confort est thermoformée.
- Au moins une partie de l'élément de confort est amovible.

D'autres caractéristiques et avantages de l'invention seront mieux compris à l'aide de la description qui va suivre, en regard des dessins annexés illustrant, selon des formes de réalisation non limitatives, comment l'invention peut être réalisée, et dans lequel :
- la figure 1 est une vue en perspective avant du masque selon une premier mode de réalisation de l'invention ;
- la figure 2 est une vue en perspective arrière du masque sans sangle ;
- la figure 3 est une vue de dessous du masque sans sangle ;
- la figure 4 est une vue arrière simplifiée du masque sans sangle ;
- la figure 5 est une vue arrière simplifiée d'un deuxième mode de réalisation ;
- la figure 6 est une vue arrière simplifiée d'un troisième mode de réalisation,

Le masque 1 comprend un châssis 2 supportant un écran de protection oculaire 3. Une sangle 4 est fixée aux extrémités latérales du châssis 2. La sangle 4 et le châssis 2 forment une boucle destinée à entourer le crâne de l'utilisateur pour assurer le maintien en place du masque.

Dans la suite de la description, il sera fait usage de termes tels que « horizontal », « vertical », « supérieur », « inférieur », « haut », « bas », « avant », « arrière », « devant », « derrière ». Ces termes doivent être interprétés en fait de façon relative en relation avec la position normale que le masque occupe sur le visage de l'utilisateur quand il se tient debout, tête droite. Par « arrière », on désigne les parties orientées côté tête / yeux de l'utilisateur.

Le châssis 2 supporte un élément de confort 5 disposé à l'arrière du châssis, à l'interface entre le châssis et le visage de l'utilisateur. L'élément de confort est destiné à venir en contact avec des zones du visage entourant les yeux.

L'élément de confort 5 comprend au moins une partie frontale 51 destinée à venir en contact avec une partie du front de l'utilisateur et une partie nasale 52 destinée à venir en contact avec une partie du nez de l'utilisateur. La partie frontale vient couvrir une zone du front située sensiblement au-dessus des sourcils. L'élément de confort appuie ainsi sur une zone « dure » du fait de la présence de l'os frontal du crâne. La partie nasale vient couvrir le dos du nez, les flancs du nez, à la limite des ailes du nez. Cette partie nasale peut également s'étendre sur une partie médiale des pommettes. Dans cette région, l'élément de confort appuie ainsi sur une zone « molle » et sensible du fait de la présence de cartilages plus fragiles que l'os crânien.

L'élément de confort peut être une pièce unitaire intégrant la partie frontale et la partie nasale. Alternativement, l'élément de confort peut comprendre plusieurs pièces distinctes, chacune étant reliée au châssis. Par exemple, la partie frontale et la partie nasale sont deux pièces distinctes.

L'élément de confort comprend au moins deux parties. Chaque partie correspond à une zone d'appui spécifique sur le visage. Chaque partie est constituée de matériau ayant des propriétés mécaniques spécifiques. Au sens de l'invention, un matériau peut être une couche mono matière ou un composite constitué de plusieurs couches de matériaux différents. Dans ce dernier cas, ce matériau correspond à un « complexage »,

L'invention se caractérise par le fait que le matériau utilisé pour la partie nasale présente des propriétés mécaniques d'écrasement différentes du matériau utilisé pour la partie frontale de sorte que la course d'écrasement maximale du matériau de la partie nasale sollicité par un effort de compression déterminé est plus importante que la course d'écrasement du matériau de la partie frontale sollicité par le même effort de compression déterminé. Autrement dit, si on exerce le même effort de compression déterminé sur la partie frontale et sur la partie nasale, la partie nasale va davantage se déformer.

L'écrasement d'une partie de l'élément de confort correspond à une compression de l'élément de confort dans son épaisseur, à savoir selon une direction horizontale traduisant la compression de l'élément de confort entre le visage et le châssis. L'effort de compression est donc un effort exercé dans une direction X orientée suivant l'épaisseur de l'élément de confort. La course d'écrasement correspond à la variation de la position de la face sur laquelle on exerce l'effort de compression selon la direction X. Cette course est égale à la différence entre la position de la face non sollicitée et la position de la face sollicitée par l'effort de compression déterminé.

Pour obtenir cette différence de course entre les deux parties, frontale et nasale, plusieurs solutions sont envisageables.

Dans un premier cas, au moins une couche de la partie nasale 52 est constituée d'une matière plus souple que la matière d'au moins une couche de la partie frontale 51. En conséquence, la matière plus souple de la partie nasale peut se déformer davantage que la matière utilisée pour la partie frontale. Par exemple, on peut utiliser des mousses ayant différentes densités. Pour la partie nasale, on utilise une mousse ayant une plus faible densité qu'une mousse utilisée pour la partie frontale. Ainsi, en étant moins dense, la mousse de la partie nasale se déforme davantage que la mousse de la partie frontale. La partie nasale et/ou frontale peut être monocouche ou multicouche. L'épaisseur de la partie nasale peut être la même ou être différente de l'épaisseur de la partie frontale.

Dans un deuxième cas, l'épaisseur d'au moins une couche de la partie nasale est supérieure à l'épaisseur d'au moins une couche de la partie frontale. En étant plus épaisse, la couche de la partie nasale peut se déformer sur une course plus importante. La matière utilisée pour la couche de la partie nasale peut être la même ou être différente de la matière utilisée pour la couche de la partie frontale. Par exemple, on peut utiliser des mousses ayant la même densité ou des densités différentes. Là aussi, la partie nasale et/ou frontale peut être monocouche ou multicouche.

Ainsi, pour ajuster les propriétés mécanique d'écrasement d'un matériau, on peut modifier la nature de la matière utilisée pour au moins une couche et/ou modifier l'épaisseur d'au moins une couche, que le matériau soit monocouche ou multicouche.

Pour réaliser une couche de la partie nasale et/ou frontale, on utilise avantageusement une mousse en PolyUréthane (PU). Alternativement, on peut envisager d'utiliser d'autres matières permettant une grande déformation pour un faible effort. Pour certaines couches, on peut aussi utiliser d'autres types de mousses comme des mousses en PolyChlorure de Vinyle (PVC), en PolyEthylène (PE), en Ethylène-Acétate de Vinyle (EVA),

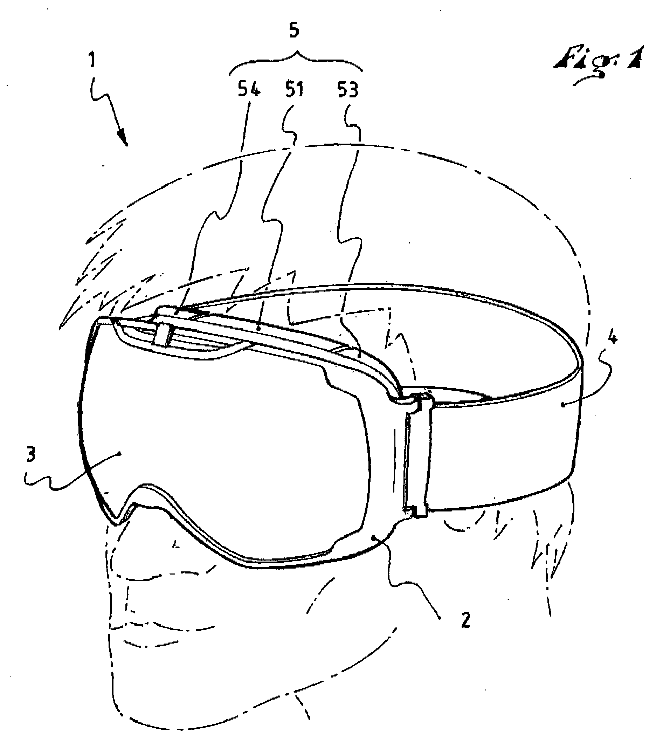

Dans l'exemple Illustré aux figures 1 à 3, l'élément de confort 5 comprend plusieurs pièces distinctes fixées sur la face arrière du châssis 2.

Une première pièce constitue la partie frontale 51, d'épaisseur e51, couvrant une partie du front de l'utilisateur. Cette partie frontale est constituée d'un matériau complexé, c'est-à-dire, comprenant plusieurs couches, Une première couche 511, d'épaisseur e511 est fixée sur la face arrière du châssis 2. Dans cet exemple, cette première couche est réalisée dans un premier type de mousse : une mousse en PolyChlorure de Vinyle. Une deuxième couche 512, d'épaisseur e512 est fixée sur la première couche 511. Elle succède à la première couche dans le sens de l'épaisseur, dans une direction d'éloignement du châssis. Dans cet exemple, cette seconde couche est réalisée dans un deuxième type de mousse : une mousse en PolyUréthane de densité de l'ordre de 20 kg/m³. Enfin, un tissu 513, de faible épaisseur, est fixé sur la seconde couche 512. Il succède à la seconde couche dans le sens de l'épaisseur. Le tissu apporte le confort de contact. Ce tissu présente avantageusement des propriétés de douceur au contact, de respirabilité et d'absorption d'humidité, pour la transpiration. L'agencement des couches est tel que l'épaisseur de la partie frontale e51 correspond à la somme des épaisseurs de la première couche e511, de la seconde couche e512 et du tissu. Le matériau de la partie frontale 51 comprend ainsi successivement, à partir du châssis 2, une première couche 511, une deuxième couche 512 et un tissu 513. Selon un mode de réalisation, l'épaisseur de la première couche e511 est comprise entre 30 et 50 % de l'épaisseur de la partie frontale e51. L'épaisseur de la seconde couche e512 est comprise entre 50 et 70 % de l'épaisseur de la partie frontale e51. Par exemple, l'épaisseur de la première couche e511 est de l'ordre de quatre millimètres et l'épaisseur de la seconde couche e512 est de l'ordre de huit millimètres. Dans la zone frontale, l'élément de confort doit assurer un bon maintien du châssis, notamment un bon maintien vertical. Pour cela, la pression de contact avec le visage doit être assez importante pour éviter que le masque ballotte ou se déplace verticalement Par ailleurs, la présence de l'os frontal permet d'accepter une pression assez importante sans désagrément. Aussi, la partie frontale de l'élément de confort ne doit pas nécessairement se déformer beaucoup mais, au contraire, permettre un bon placage avec une pression adaptée. Il est alors préférable d'avoir un bon retour élastique de cette partie frontale. Pour cela, ici, la mousse utilisée pour la première couche 511 présente un module d'élasticité nettement plus élevé que la mousse utilisée pour la seconde couche 512. Le confort est apporté par la seconde couche, le maintien est apporté par la « nervosité » de la première couche.

Une deuxième pièce constitue la partie nasale 52, d'épaisseur e52 couvrant une partie du nez de l'utilisateur. Cette partie nasale est constituée d'un matériau complexé, c'est-à-dire, comprenant plusieurs couches. Une première couche 521, d'épaisseur e521 est fixée sur la face arrière du châssis 2. Dans cet exemple, cette première couche est réalisée dans un premier type de mousse : une mousse en en PolyUréthane de densité de l'ordre de 60 kg/m³, Une deuxième couche 522, d'épaisseur e522 est fixée sur la première couche 521. Elle succède à la première couche dans le sens de l'épaisseur, dans une direction d'éloignement du châssis. Dans cet exemple, cette seconde couche est réalisée dans un deuxième type de mousse : une mousse en PolyUréthane de densité de l'ordre de 20 kg/m³. Enfin, un tissu 523, de faible épaisseur, est fixé sur la seconde couche 522. Il succède à la seconde couche dans le sens de l'épaisseur. Le tissu apporte le confort de contact. Ce tissu présente principalement des propriétés de douceur au contact. L'agencement des couches est tel que l'épaisseur de la partie nasale e52 correspond à la somme des épaisseurs de la première couche e521, de la seconde couche e522 et du tissu. Le matériau de la partie nasale 52 comprend ainsi successivement, à partir du châssis 2, une première couche 521, une deuxième couche 522 et un tissu 523. Selon un mode de réalisation, l'épaisseur de la première couche e521 est comprise entre 10 et 30 % de l'épaisseur de la partie nasale e52. L'épaisseur de la seconde couche e522 est comprise entre 70 et 90 % de l'épaisseur de la partie nasale e52. Par exemple, l'épaisseur de la première couche e521 est de l'ordre de quatre millimètres et l'épaisseur de la seconde couche e522 est de l'ordre de douze millimètres. Dans la zone nasale, l'élément de confort doit assurer une bonne adaptabilité à la morphologie du visage et un bon confort. Pour cela, la partie nasale doit pouvoir se déformer fortement pour épouser les formes du visage recouvertes avec un minimum de pression de contact. En effet, les cartilages présents dans cette zone rendent cette partie du visage sensible. Il est donc préférable de réduire la pression exercée par le masque sur cette zone du visage. Cela permet aussi de dégager les narines pour faciliter la respiration de l'utilisateur. Le confort est principalement apporté par la seconde couche. La première couche contribue également au confort, permet un amortissement progressif et apporte un peu d'élasticité pour le maintien.

Selon ce mode de réalisation, l'épaisseur de la partie nasale e52 est supérieure à l'épaisseur de la partie frontale e51. Par exemple, l'épaisseur de la partie frontale e51 est comprise entre 60 et 80 % de l'épaisseur de la partie nasale e52. Pour cette illustration, la variation d'épaisseur est essentiellement réalisée par la variation d'épaisseur de la seconde couche e512/e522.

Dans le mode de réalisation illustré, l'élément de confort comprend deux autres pièces distinctes.

Une troisième pièce constitue la partie latérale gauche 53, d'épaisseur e53, couvrant la tempe gauche et la pommette gauche du visage de l'utilisateur. Cette partie latérale gauche relie l'extrémité gauche de la partie frontale à l'extrémité gauche de la partie nasale. Cette partie latérale gauche est constituée d'un matériau complexé, c'est-à-dire, comprenant plusieurs couches. Une première couche 531, d'épaisseur e531 est fixée sur la face arrière du châssis 2. Dans cet exemple, cette première couche est réalisée dans un premier type de mousse : une mousse en Polyuréthane de densité de l'ordre de 60 kg/m³. Une deuxième couche 532, d'épaisseur e532 est fixée sur la première couche 531. Elle succède à la première couche dans le sens de l'épaisseur, dans une direction d'éloignement du châssis. Dans cet exemple, cette seconde couche est réalisée dans un deuxième type de mousse : une mousse en PolyUréthane de densité de l'ordre de 20 kg/m³. Enfin, un tissu 533, de faible épaisseur, est fixé sur la seconde couche 532. Il succède à la seconde couche dans le sens de l'épaisseur, à partir du châssis. Le tissu apporte le confort de contact. Ce tissu présente principalement des propriétés de douceur au contact. L'agencement des couches est tel que l'épaisseur de la partie latérale gauche e53 correspond à la somme des épaisseurs de la première couche e531, de la seconde couche e532 et du tissu. Le matériau de la partie latérale gauche 53 comprend ainsi successivement, à partir du châssis 2, une première couche 531, une deuxième couche 532 et un tissu 533. Selon un mode de réalisation, l'épaisseur de la première couche e531 est comprise entre 30 et 50 % de l'épaisseur de la partie latérale gauche e53. L'épaisseur de la seconde couche e532 est comprise entre 50 et 70 % de l'épaisseur de la partie latérale gauche e53. Par exemple, l'épaisseur de la première couche e531 est de l'ordre de quatre millimètres et l'épaisseur de la seconde couche e532 est de l'ordre de huit millimètres.

Une quatrième pièce constitue la partie latérale droite 54, d'épaisseur e54, couvrant la tempe droite et la pommette droite du visage de l'utilisateur. Cette partie latérale droite 54 est symétriquement identique à la partie latérale gauche 53. Elle est construite de manière analogue et présente les mêmes caractéristiques. Elle comprend une première couche 541, d'épaisseur e541, une seconde couche 542, d'épaisseur e542 et un tissu 643.

Dans cet exemple, l'épaisseur des parties latérales e53, e54 est inférieure à l'épaisseur de la partie nasale e52 et sensiblement égale à l'épaisseur de la partie frontale e51, Avantageusement, le matériau utilisé pour les parties latérales 53, 54 présentent des propriétés mécaniques d'écrasement intermédiaire par rapport aux matériaux utilisés pour les parties nasale et frontale. Alternativement, le matériau utilisé pour les parties latérales 53, 54 peut présenter des propriétés mécaniques d'écrasement analogues au matériau utilisé pour une des parties nasale ou frontale.

Cette construction de l'élément de confort en parties distinctes permet de changer facilement les parties endommagées ou usées. Cela permet également d'ajuster le confort de l'utilisateur en augmentant ou en réduisant la pression de contact dans certaines zones d'appui sur le visage. D'autre part, cela permet d'améliorer l'adaptation à la morphologie du visage. Par exemple, en fonction de la taille du nez, on peut utiliser une partie nasale ayant une épaisseur adaptée. Pour faciliter cette interchangeabilité, on peut prévoir que les parties distinctes sont montées amovibles sur le châssis, Les moyens d'accroches peuvent être de type crochet (VELCRO®), bouton, clip...

Dans ces exemples, l'élément de confort 5 forme une bande de largeur sensiblement constante, comprise entre dix et vingt millimètres. La bande constitue une boucle « fermée », entourant les yeux, qui se présente sous la forme générale d'un rectangle horizontal. L'épaisseur de la bande peut varier conformément aux modes de réalisation décrits précédemment. Dans sa partie inférieure, la bande forme un « V » inversé pour dégager les ailes du nez afin de ne pas gêner la respiration.

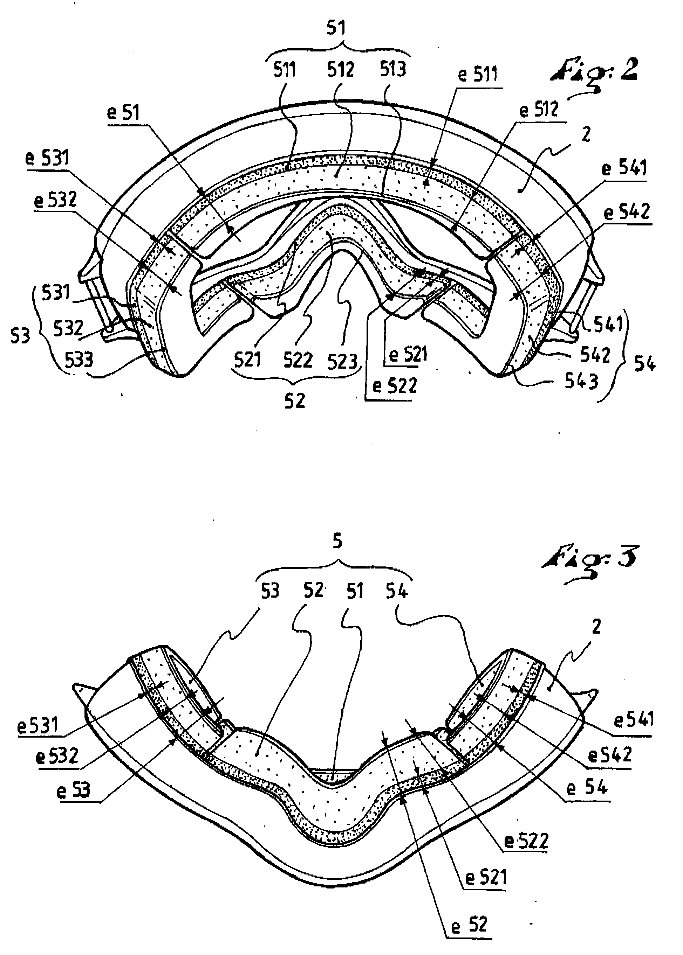

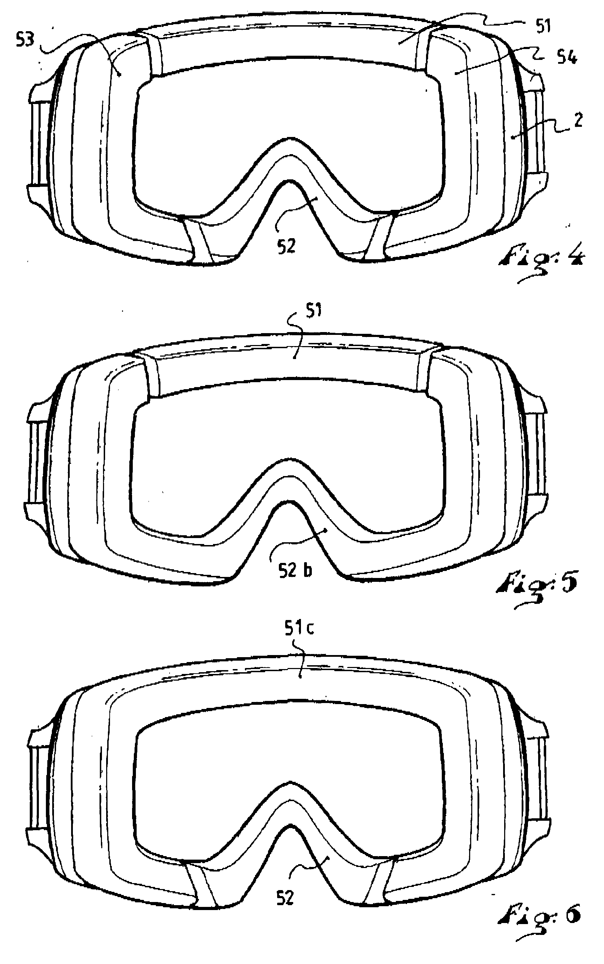

La figure 4 est une vue arrière simplifiée du masque illustré aux figures 2 et 3 dans le sens où les couches des parties constitutives 51, 52, 53 et 54 ne sont pas représentées.

L'élément de confort n'est pas nécessairement composé de quatre pièces distinctes, il peut comprendre plus de pièces ou moins de pièces. Par exemple, l'élément de confort peut ne comprendre que deux éléments.

Les figures 5 à 6 illustrent deux autres modes de réalisation d'un masque équipé d'un élément de confort comprenant deux éléments. Ces figures sont des représentations simplifiées dans le sens où les couches des parties constitutives ne sont pas représentées.

La figure 5 Illustre un deuxième mode de réalisation dont l'élément de confort comprend une partie frontale 51 analogue à celle décrite précédemment et une partie inférieure 52b couvrant une partie du nez, les pommettes et les tempes. La partie inférieure correspond sensiblement à la combinaison de la partie nasale avec les deux parties latérales du premier mode de réalisation. Ces trois parties distinctes forment une même pièce continue. Dans ce mode de réalisation, l'épaisseur de la partie inférieure peut être variable, par exemple plus épaisse au niveau du nez.

La figure 6 illustre un troisième mode de réalisation dont l'élément de confort comprend une partie frontale 51c couvrant une partie du front, les pommettes et les tempes et une partie nasale 52 analogue à celle décrite précédemment. La partie supérieure correspond sensiblement à la combinaison de la partie frontale avec les deux parties latérales du premier mode de réalisation. Ces trois parties distinctes forment une même pièce continue.

Alternativement, la démarcation entre les différentes zones d'appui sur le visage peut être localisée différemment. Par exemple, la zone frontale peut couvrir les tempes et s'arrêter au milieu d'un bord latéral.

Dans les modes de réalisation décrits, le matériau utilisé pour les parties de l'élément de confort est composé de deux couches. Alternativement, le matériau peut comprendre davantage de couches, par exemple, une succession de mousses dont la densité varie progressivement. Cela permet d'avoir une bonne adaptabilité morphologique et un amortissement/déformation progressif, ce qui améliore le confort de portage.

Selon un autre mode de réalisation, le matériau est monocouche.

En variante, le nombre de couches entre les différentes parties de l'élément de confort n'est pas le même. Par exemple, on peut avoir un matériau monocouche pour la partie nasale et un matériau ayant deux couches pour la partie frontale, ou inversement.

Dans une autre solution alternative, l'élément de confort comprend une première couche continue commune pour les différentes parties constitutives mais une seconde couche spécifique à chaque partie constitutive. Cette construction permet d'avoir un élément de confort unitaire plus facile à manipuler lorsqu'il n'est pas assemblé au châssis.

Les parties de l'élément de confort peuvent être assemblées au châssis par collage, soudage ou par des moyens d'accroche amovible.

Dans un autre mode de réalisation, les couches et/ou parties de l'élément de confort peuvent être réalisées par bi-injection ou surmoulage, ce qui permet d'avoir une pièce unitaire.

Dans une autre variante, l'élément de confort est réalisé par thermoformage à partir d'une mousse continue s'étendant autour des yeux. L'opération de thermoformage permet, par exemple, de faire varier l'épaisseur de la mousse en fonction des zones d'appui. Ainsi, on peut obtenir une zone plus épaisse dans la partie nasale de l'élément de confort et plus fine dans la partie frontale de l'élément de confort. La réduction de l'épaisseur, localement, par thermoformage, permet une modification de la densité de la mousse dans cette zone.

Alternativement, l'élément de confort peut comprendre plusieurs parties distinctes thermoformées destinées à être solidarisées avec le châssis du masque.

Bien entendu, d'autres valeurs de densité de mousse peuvent être envisagées que celles décrites précédemment.

L'invention n'est pas limitée à ces modes de réalisation. Il est possible de combiner ces modes de réalisation.

L'invention n'est pas limitée aux modes de réalisation précédemment décrits mais s'étend à tous les modes de réalisation couverts par les revendications annexées.

### REFERENCES

1. Masque
2. Châssis
3. Ecran
4. Sangle
5. Elément de confort
   51. Partie frontale
      511. Première couche
      512. Deuxième couche
      513. Textile
   52. Partie nasale
      521. Première couche
      522. Deuxième couche
      523. Textile
   53. Partie latérale gauche 531. Première couche 532. Deuxième couche 533. Textile
   54. Partie latérale droite
      541. Première couche
      542. Deuxième couche
      543. Textile

## Revendications

1. Masque de protection oculaire (1) pour la pratique de sports en plein air comprenant :
- un écran de protection oculaire (3),
- un châssis (2) supportant l'écran,
- un élément de confort (5) solidaire du châssis, l'élément de confort comprenant une partie nasale (52) destinée à venir en contact avec une partie du nez de l'utilisateur et une partie frontale (51) destinée à venir en contact avec une partie du front de l'utilisateur,
**caractérisé en ce que**
le matériau utilisé pour la partie nasale présente des propriétés mécaniques d'écrasement différentes du matériau utilisé pour la partie frontale de sorte que la course d'écrasement maximale du matériau de la partie nasale sollicité par un effort de compression déterminé est plus importante que la course d'écrasement du matériau de la partie frontale sollicité par le même effort de compression déterminé.

2. Masque de protection oculaire (1) selon la revendication 1, **caractérisé en ce qu'**au moins un matériau constituant les parties nasale et/ou frontale est composite, le matériau comprenant alors plusieurs couches (511, 512, 521, 522) de matières différentes.

3. Masque de protection oculaire (1) selon la revendication précédente, **caractérisé en ce que** le matériau de la partie frontale et/ou le matériau de la partie nasale comprend successivement, à partir du châssis (2), une première couche (511, 521) de mousse d'un premier type et une seconde couche (512, 522) de mousse d'un deuxième type, la densité de la mousse de la première couche étant plus forte que la densité de la mousse de la seconde couche.

4. Masque de protection oculaire (1) selon la revendication précédente, **caractérisé en ce que** l'épaisseur de la première couche (e521) de la partie nasale (52) est comprise entre 10 et 30 % de l'épaisseur de la partie nasale (e52) et **en ce que** l'épaisseur de la seconde couche (e522) de la partie nasale (52) est comprise entre 70 et 90 % de l'épaisseur de la partie nasale (e52).

5. Masque de protection oculaire (1) selon la revendication précédente, **caractérisé en ce que** l'épaisseur (e522) de la seconde couche de la partie nasale est supérieure à l'épaisseur (e512) de la seconde couche de la partie frontale.

6. Masque de protection oculaire (1) selon la revendication précédente, **caractérisé en ce que** les deux secondes couches (512, 522) sont constituées du même type de mousse.

7. Masque de protection oculaire (1) selon la revendication 3 ou 4, **caractérisé en ce que** la densité de la mousse de la seconde couche (522) de la partie nasale est inférieure à la densité de la mousse de la seconde couche (512) de la partie frontale.

8. Masque de protection oculaire (1) selon l'une des revendications 3 à 6, **caractérisé en ce que** la mousse de la première couche (521) de la partie nasale est de nature différente de la mousse de la première couche (511) de la partie frontale.

9. Masque de protection oculaire (1) selon l'une des revendications précédentes, **caractérisé en ce que** l'épaisseur (e52) de la partie nasale est supérieure à l'épaisseur (e51) de la partie frontale.

10. Masque de protection oculaire (1) selon l'une des revendications précédentes, **caractérisé en ce que** l'élément de confort comprend deux parties latérales (53, 54), chaque partie latérale étant destinée à venir en contact avec la tempe et la pommette d'un côté du visage de l'utilisateur, chaque partie latérale reliant une extrémité de la partie frontale à une extrémité de la partie nasale, le matériau utilisé pour les parties latérales présentant des propriétés mécaniques d'écrasement intermédiaire par rapport aux matériaux utilisés pour les parties nasale et frontale.

11. Masque de protection oculaire (1) selon l'une des revendications précédentes, **caractérisé en ce que** le nombre de couches (511, 512, 521, 522, 531, 532, 541, 542) de matière entre les différentes parties (51, 52, 53, 54) de l'élément de confort (5) est différent.

12. Masque de protection oculaire (1) selon l'une des revendications précédentes, **caractérisé en ce que** l'élément de confort (5) comprend au moins deux pièces distinctes, une première pièce (51) formant la partie nasale, la deuxième pièce (52) formant la partie frontale.

13. Masque de protection oculaire (1) selon la revendication précédente, **caractérisé en ce que** l'élément de confort (5) comprend quatre pièces distinctes: une partie frontale (51), une partie nasale (52) et deux parties latérales (53, 54).

14. Masque de protection oculaire (1) selon l'une des revendications précédentes, **caractérisé en ce qu'**au moins une partie (51, 52, 53, 54) de l'élément de confort (5) est thermoformée.

15. Masque de protection oculaire (1) selon l'une des revendications précédentes, **caractérisé en ce qu'**au moins une partie (51, 52, 53, 54) de l'élément de confort (5) est amovible.
